# EUROPEAN PATENT APPLICATION

(11) **EP 4 710 998 A2**
(43) Date of publication of application: **18.03.2026**
(21) Application number: 26154139.5
(22) Date of filing: 05.07.2022
(51) Int. Cl.: A61N 5/06

(54) **PHOTOTHERAPEUTIC APPARATUS**

(30) Priority: 05.07.2021 EP 21183665
(62) Divisional of application: 22744176.3
(71) Applicant: Danmarks Tekniske Universitet, 2800 Kongens Lyngby (DK); Optoceutics ApS, 1600 København V (DK)
(72) Inventor: BROENG, Jes, 2800 Kgs. Lyngby (DK); CARSTENSEN, Marcus Schultz, 2800 Kgs. Lyngby (DK); CARRILLO, Gustavo Feijóo, 2800 Kgs. Lyngby (DK); NGUYEN, Ngoc Mai, 2800 Kgs. Lyngby (DK)
(74) Representative: Guardian IP Consulting I/S

(57) **Abstract**

A phototherapeutic apparatus comprising a plurality of light sources and a control circuit, the plurality of light sources being arranged in a spatial pattern so as to form a display having a display area and each of the plurality of light sources being individually controllable to emit colored light; wherein the control circuit is configured to: receive image data; control the plurality of light sources to emit a spatially resolved pattern of emitted colored light, the spatially resolved pattern representing the received image data, wherein each of the plurality of light sources emits colored light perceivable by a human observer as having respective user-perceptible target image colors; wherein the control circuit is configured to control at least each of a first subset of the plurality of light sources to alternatingly emit respective first and second light, alternating at a color-flicker frequency; the first light having a first set of color components resulting in a first emitted color and the second light having a second set of color components resulting in a second emitted color; wherein the color-flicker frequency is selected high enough to cause the alternatingly emitted first and second light to be perceived by a human observer as light having a user-perceptible fused color corresponding to the corresponding target image color.

## Description

### TECHNICAL FIELD

The present invention relates to a phototherapeutic apparatus, in particular, a phototherapeutic apparatus for light-induced gamma brain stimulation for preventing, slowing, postponing, and/or treating psychiatric and neurological diseases such as Alzheimer's disease, different types of dementia, sleep, anxiety, or other circadian rhythm related disorders, and to situations wherein gamma brain stimulation is paramount for a desired outcome, such as increasing, inducing or restoring a particular amount of neuronal activity of the neuronal network of the brain.

### BACKGROUND

The use of light-induced gamma brain stimulation has been suggested for various therapeutic and/or preventive applications and/or cognitive training.

WO 2018/152255 discloses a light therapy system (e.g., phototherapy device), such as for treatment of Alzheimer's disease, depression, dementia, short-term memory, or for improved learning, improved athletic performance, or improved cognitive performance. This prior art light system comprises a blue light source that operates at a frequency in the range from 20 to 50 Hz (preferably around 40 Hz), whereby the system enables retinal ganglion cells of a human to be exposed in order to stimulate brain waves (gamma oscillations in the human's brain).

However, it remains desirable to provide a phototherapeutic apparatus that is versatile in use and facilitates exposure of users over an extended period of time in a user-friendly manner.

### SUMMARY

According to one aspect, disclosed herein are embodiments of a phototherapeutic apparatus comprising a plurality of light sources and a control circuit, the plurality of light sources being arranged in a spatial pattern so as to form at least a part of a display area of a display and each of the plurality of light sources being individually controllable to emit colored light; wherein the control circuit is configured to:
- receive image data representing, for each light source of the plurality of light sources, a respective target image color to be represented by said light source;
- control each light source of the plurality of light sources to emit colored light perceivable by a human observer as having the target image color to be represented by said light source;
wherein the control circuit is configured, for each light source of at least a first subset of the plurality of light sources, to:
- determine, responsive to the target image color to be represented by said light source, a first and a second set of color components; and to
- control said light source to alternatingly emit at least respective first and second light, alternating at a color-flicker frequency; the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source.

Accordingly, embodiments of the apparatus disclosed herein provide stimulation of neural activity in a subject's brain, in particular stimulation of gamma waves, by multiple light-emitting pixels or other light sources arranged in a spatial pattern.

Generally, the apparatus comprises a display having a display area. The plurality of light sources are arranged in a spatial pattern so as to form the display area. In one embodiment, the apparatus comprises a display comprising an array of individually addressable pixels, i.e., the plurality of light sources form respective pixels of the display. The display may be an LED display, an OLED display, a Q-LED display, an LCD, or a display utilizing another suitable display technology.

Embodiments of the apparatus described herein have widespread use in real-life applications and are suitable for use in daily lives, e.g., in the form of a television set or other display. The apparatus displays a spatially resolved pattern of emitted colored light, e.g., in the form of an image, graphic pattern, or otherwise a pattern that varies across the display area of the display. At least in some embodiments, the image data is time-varying image data. The apparatus may thus be configured to display a time-varying series of spatially resolved patterns of emitted colored light, e.g., in the form of a stream of image frames representing a video, film, graphical animation, or otherwise a pattern that perceptibly varies over time and across the display area of the display. The light sources emitting alternating colored light perceivable as a fused color will also be referred to as color fusion pixels. At least some embodiments of the apparatus implement imperceptible gamma stimulation. The apparatus implements display technology that includes color fusion pixels, i.e., pixels that alternatingly emit first and second colored light at a color-flicker frequency high enough to cause the alternatingly emitted first and second light to be perceived by a human observer as light having a user-perceptible fused color.

In some embodiments, the color-flicker frequency is selected smaller than a critical flicker-fusion frequency (CFF) and higher than a critical color-fusion frequency (CCFF).

The "critical flicker-fusion frequency" refers to a concept in the psychophysics of vision. It is defined as the frequency at which an intermittent (e.g., blinking) light stimulus appears to be completely steady to the average human observer. Flicker fusion threshold is related to persistence of vision. The critical flicker-fusion frequency depends on the luminance of the stimulus and its size (see, e.g., Hecht and Smith (1936) J. Gen. Physiol. 19(6): 979-89). For a large, high luminance stimulus covering the fovea, like a full screen white field on a CRT, flicker fusion occurs at about 60 Hz.

The phrase "a color-flicker frequency high enough to cause the alternatingly emitted first and second light to be perceived by a human observer as light having a user-perceptible fused color" when used with respect to a flickering or blinking light of alternating colors means that a human illuminated by or observing the illumination cannot see the blinking/flickering component of the light and instead perceives the illumination as substantially constant, even where the frequency of the blinking light/light component is below the critical flicker-fusion frequency. The threshold frequency above which the alternatingly emitted first and second light is perceived by a human observer as light having a user-perceptible fused color is also referred to as critical color-fusion frequency (CCFF).

Accordingly, users of the apparatus can be exposed to the emitted light over extended periods of times, e.g., while watching a movie, images, graphic patterns, while performing tasks involving a graphical user interface of a computer or other electronic device, or the like, without the user getting unduly tired, bored, or otherwise experiencing the light therapy as unduly annoying. The ability to provide exposure over a long period of time improves the stimulating effect of the light. Particularly, at least some embodiments of the apparatus provide an effective dosage of brain-stimulating light while the user of the apparatus can watch colorful videos and images through modern and novel canonical display technology.

Embodiments of the apparatus may be used for various light therapy and/or other brain stimulation applications. For example, embodiments of the apparatus may be used for treatment and/or prevention of Alzheimer's disease, depression, dementia, short-term memory, or for improved learning, improved athletic performance, or improved cognition. For example, as many people use extended periods of time in front of a display screen, e.g., a TV, computer, smartphone, embodiments of the present apparatus allow the integration of light therapy and/or light stimulation into existing daily routines.

In some embodiments, the apparatus is configured to display a spatial pattern that changes over time, e.g., changes at a spatial flicker frequency, i.e., new patterns may be presented at said spatial flicker frequency. Examples of spatial patterns that change over time include a moving grating, a rotating pattern, e.g., a rotating patterned disk, or a square pattern. The spatial flicker frequency may be between 5- 60 Hz, such as between 25 Hz and 60 Hz, such as between 30 Hz and 50 Hz, such as between 35 Hz and 45 Hz, such as between 38 Hz and 42 Hz, such as 40 Hz. Accordingly, the apparatus enables the eyes to be exposed to spatially varying patterns to stimulate brain waves (preferable gamma oscillations in the brain).

According to some embodiments, the color-flicker frequency is selected for invoking a therapeutically effective neural response in the human observer's brain. According to some embodiments, the alternatingly emitted light at said color-flicker frequency is selected to stimulate or to entrain brain waves, in particular gamma oscillations, in a human's brain when the human is exposed to said alternatingly emitted light. In particular, in some embodiments, the color-flicker frequency is between 5 Hz and 60 Hz, such as between 25 Hz and 60 Hz, such as between 30 Hz and 50 Hz, such as between 35 Hz and 45 Hz, such as between 38 Hz and 42 Hz, such as 40 Hz.

The image data may represent one or more captured pictures and/or computer-generated graphics or other computer-generated image data. In some embodiments, the control circuit is configured to control the plurality of light sources so as to change the displayed image over time. In particular, the control circuit may be configured to receive a stream of image data representing a video and to control the plurality of light sources to display the video within the display area. Accordingly, the apparatus may display a video or other image content that changes over time, thereby facilitating the use of the apparatus over an extended period of time, as the display may display entertaining and/or informative content, such as TV programs, movies, or the like. Accordingly, the user may be exposed to the flickering light during everyday activities like watching television or the like.

In some embodiments, the displayed image data may include high-resolution images, while other embodiments display simple geometric patterns. For example, in one embodiment, the control circuit is configured to control the plurality of light sources so as to cause a simulated movement of a first light-emitting area emitting light having a first user-perceptible target color relative to a second light-emitting area emitting light having a second user-perceptible target color. In a particular embodiment, the apparatus displays a moving grading, e.g., a spatial pattern including multiple segments, that are updated at a spatial flicker frequency such that they appear to move across at least a part of the display area. The segments may be arranged in a grid or other regular pattern. The segments may be illuminated in different colors, which may be visually distinguishable or visually indistinguishable. Accordingly, a cost-efficient apparatus may be provided where the display may have a relatively low spatial resolution and where the number of displayable target colors may be limited.

In some embodiments, the first and second user-perceptible target colors are visually indistinguishable for a human observer but have different spectral distributions. An example of visually indistinguishable colors may be two whites having respective spectral distribution. An example of a pair of visually distinguishable colors include read and green.

It will be appreciated that different embodiments of the apparatus may include different numbers of light sources, in particular displays of different resolution. It will be appreciated that the number of light sources and the density of light sources across the display area may depend on the display technology, the dimensions of the display, and/or other factors such as the desired quality of images. In some embodiments, the number of light sources is selected such that a desired resolution, e.g. HD, full HD, 4K or other suitable resolution, is achieved. The dimensions of the display area may vary, including, but not limited to display areas ranging from a smartphone display to a large television screen, e.g. a 200 inch television screen. It will further be appreciated that the plurality of light sources may form the entire display area of a display or only a part of the display area of the display, e.g. only a subarea.

In some embodiments, all light sources of the display, in particular all pixels of the display, may be operable and controllable to emit respective target image colors as respective fused colors by alternatingly emitting respective first and second colors. In other embodiments, only a subset of the light sources may be so adapted while the remaining light sources represent conventional display pixels. In some embodiments, some or all light sources of the display, in particular some or all pixels of the display, may be selectively operable and selectively controllable to emit respective target image colors by conventional color mixing - in particular by concurrently emitting respective color components that mix to the intended target image color - or as respective fused colors - i.e. by alternatingly emitting respective first and second colors. The first subset of light sources may partly or completely be predetermined. Additionally or alternatively, the control circuit may select the first subset of the light sources that are to be operated as a color fusion pixels, i.e. operated to display colors as fused colors, based on one or more criteria, e.g. responsive to a desired strength of the therapeutic neural stimulation, responsive to a user-control and/or responsive to one or more properties of the received image data. In some embodiments, the plurality of light sources of the display is divided (in a predetermined manner or adaptively by the control circuit) into the first subset and a second subset, disjoint from the first subset, and the control circuit is configured to only operate the light sources of the first subset as color fusion pixels, i.e. to alternatingly emit respective color components at the color-flicker frequency, i.e. to display respective target colors by alternatingly emitting respective color components that are perceived as the target color when being color-fused by being alternatingly emitted at the color-flicker frequency. The second subset may be operated to emit respective target image colors only by concurrently emitting respective color components that mix to be perceived as the target color.

In some embodiments, the control circuit is configured to determine, responsive to the received image data, first and second sets of color components for respective ones of the first subset of said plurality of light sources; and to control each of the first subset of light sources to alternatingly emit respective first and second light having the determined respective first and second sets color components. It will be appreciated that the target image colors to be emitted by the respective light sources of the first subset may, depending on the image data to be displayed, be different from light source to light source. Accordingly, the respective first and second sets of color components determined by the control circuit may also differ from light source to light source. It will be appreciated that the light sources of the second subset may be controlled in the same manner, but with the first and second sets of color components being identical, thus effectively being operated as a conventional display pixel.

Preferably, the control circuit is configured to control at least each of the first subset of the plurality of light sources to alternatingly emit respective first and second light in a synchronized manner, in particular at the same color-flicker frequency and, preferably the same phase. In particular, it is preferred that the control circuit is configured to control all light sources of the plurality of light sources, which are alternatingly emitting respective light at a color-flicker frequency, in a synchronized manner, in particular at the same color-flicker frequency and, preferably the same phase. Accordingly, the color-flicker frequency of all color fusion pixels of the display is uniform across the display area. The present inventors have realized that it is advantageous to stimulate a user's brain by multiple light emitting pixels that are synchronized, in particular so as to provide temporal and/or spatial stimulations.

Depending on the display technology used, e.g. depending on the number of individual colored light sources each of the image pixels of a display is composed of, not all target colors may be equally well be reproducible by color fusion of alternatingly emitted color components. Accordingly, in some embodiments, the control circuit is configured to select the first subset of light sources responsive to the received image data. In particular, in some embodiments, the control circuit is configured to only include selected light sources of the plurality of light sources in the first subset, wherein the target image colors to be represented by the selected light sources belong to a predetermined set of target image colors. The remaining light sources may thus be included in a second subset of light sources that are controlled to be operated as conventional display pixels.

When displaying time-varying images, e.g. a video stream or other series of image frames, areas of rapidly changing colors may not be suitable for being displayed as color fusion pixels, as this may diminish the strength of the induced neural activity and/or diminish the color perception by the user. Accordingly, in some embodiments, the received image data is time varying image data representing, for each light source of the plurality of light sources, a series of respective target image colors; and the control circuit is configured to change the first subset responsive to the time-varying image data. Accordingly, the control circuit may select different sets of light sources to be operated as color fusion pixels at different times, in particular responsive to varying properties of the image stream to be displayed. To this end, the control circuit may employ a forward-looking strategy in order to determine how soon the image target colors to be represented by respective light sources will change. To this end, the control circuit may maintain a buffer of a suitable length, wherein the received series of image data is buffered prior to being displayed so as to allow analysis of the target image colors to be displayed. It will be appreciated that, in some embodiments, the image data may include suitable metadata including information about how long respective target colors will persist.

In particular, in some embodiments, the control circuit is configured to only include selected light sources of the plurality of light sources in the first subset, wherein the target image colors to be represented by the selected light sources persist for at least a minimum period of time, in particular at least 100 ms, such as at least 200 ms. The remaining light sources may thus be included in a second subset of light sources that are controlled to be operated as conventional display pixels. It will be appreciated that, in some embodiments, the control circuit may select the first subset of light sources based both on the target color and on the time during which the target image color of an image pixel remains constant or, at least substantially constant, e.g. only varies within a predetermined limit.

According to some embodiments, the phototherapeutic apparatus further comprises an audio output device and is configured to output a sound signal modulated at said color-flicker frequency, thereby providing additional stimulation.

In some embodiments, the control circuit is configured to determine the first and second sets of color components from a look-up table, the look-up table mapping each of a plurality of target colors to respective first and second sets of color components. Accordingly, a fast determination of the sets of color components is achieved.

In some embodiments, the control circuit is configured to control the plurality of light sources to emit the first and second light at substantially the same perceived luminance, i.e. such that the first light has a first luminance and the second light has a second luminance substantially equal to the first luminance, in particular such the any difference in luminance is sufficiently small for it to be imperceptible by the user. In particular, in some embodiments, the control circuit is configured to control the plurality of light sources such that the first light and the second light have the same lux level as perceived by an observer, in particular by an observer at a predetermined distance and/or at a distance larger than a threshold distance, such as at a distance larger than 20 cm, such as larger than 50 cm, such as larger than 1 m, such as larger than 2 m. Preferably, the first light and the second light are emitted in the same direction and/or have the same emission directional pattern in steradian.

The first and second sets of color components are selected to cause the alternatingly emitted first and second light to be perceived by a human observer as light having a user-perceptible fused color corresponding to the target image color to be represented by the light source when the first and second light are emitted at substantially the same luminance. Accordingly, the control circuit may be configured to control each light source of at least the first subset of the plurality of light sources to emit the respective first and second light at substantially the same luminance, whereby an improved color reproduction is achieved.

In some embodiments, each light source comprises a set of three or more, preferably four or more, such as five or more, light emitting sub-elements configured to emit light having respective colors. Accordingly, each of the color components of the first and second sets of color components may be selected to be a color of one of the set of light emitting sub-elements. When the first or second set of color components includes multiple color components, e.g. multiple primary colors, the corresponding first or second color is a mix of said multiple color components. A larger number of light-emitting sub-elements thus allows a larger variability of selecting first and second sets of color components and, hence, of creating different first and second colors and, consequently, of different fused colors.

The present disclosure relates to different aspects including the apparatus described above and in the following, corresponding systems, methods, and/or products, each yielding one or more of the benefits and advantages described in connection with one or more of the other aspects, and each having one or more embodiments corresponding to the embodiments described in connection with one or more of the other aspects and/or disclosed in the appended claims.

In particular, according to one aspect, disclosed herein are embodiments of a method for controlling a phototherapeutic apparatus, in particular a phototherapeutic apparatus as disclosed herein, the phototherapeutic apparatus comprising a plurality of light sources, the plurality of light sources being arranged in a spatial pattern so as to form at least a part of a display area of a display and each of the plurality of light sources being individually controllable to emit colored light, wherein the method comprises:
- receiving image data representing, for each light source of the plurality of light sources, a respective target image color to be represented by said light source;
- controlling each light source of the plurality of light sources to emit colored light perceivable by a human observer as having the target image color to be represented by said light source;

wherein controlling comprises:
   - determining, responsive to the target image color to be represented by said light source, a first and a second set of color components; and to
controlling said light source to alternatingly emit respective first and second light, alternating at a color-flicker frequency, the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source.

According to another aspect, disclosed herein are embodiments of a method for invoking gamma waves in a subject's brain, the method comprising:
- receiving image data representing, for each light source of a plurality of light sources, a respective target image color to be represented by said light source;
- controlling each light source of the plurality of light sources to emit colored light perceivable by a human observer as having the target image color to be represented by said light source;

wherein controlling comprises:
   - determining, responsive to the target image color to be represented by said light source, a first and a second set of color components; and to
controlling said light source to alternatingly emit respective first and second light, alternating at a color-flicker frequency, the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source and wherein the color-flicker frequency is selected for invoking gamma waves in the human observer's brain.

In some embodiments, the user may be positioned in front of the screen such that the user's eyes are located at a distance from the display that may e.g. be between 1.5 to 2.5 times the diagonal of the display area. The user may be positioned such that the user can observe the display at a suitable viewing angle, e.g. at about a 30-degree viewing angle or a different suitable viewing angle. For example, for a 40-inch display, the user may sit at a distance between 1.5 m and 2.5 m from the display. However, different users may prefer different distances and/or viewing angles, e.g. up to approximately 10 m.

### BRIEF DESCRIPTION OF THE DRAWINGS

Preferred embodiments will be described in more detail in connection with the appended drawings, where
FIG. 1 schematically shows an embodiment of a phototherapeutic apparatus.
FIG. 2 schematically shows a display of an embodiment of a phototherapeutic apparatus.
FIG. 3 schematically shows a light source of an embodiment of a phototherapeutic apparatus.
FIGs. 4 and 5 schematically illustrate color fusion with an embodiment of a phototherapeutic apparatus.
FIG. 6 schematically illustrates a process of controlling an embodiment of a phototherapeutic apparatus.
FIG. 7 schematically illustrates operation of an embodiment of a phototherapeutic apparatus.
FIG. 8 schematically shows a light source of another embodiment of a phototherapeutic apparatus.
FIGs. 9 - 10 schematically illustrate color fusion with an embodiment of a phototherapeutic apparatus.
FIG. 11 illustrates an example of a process for obtaining a first and a second sect of color components for a target image color.
FIG. 12 illustrates another example of a process for obtaining a first and a second sect of color components for a target image color.
FIG. 13 schematically illustrates an example of a limited color gamut.
FIG. 14 schematically illustrates a display of an embodiment of a phototherapeutic apparatus.

### DETAILED DESCRIPTION

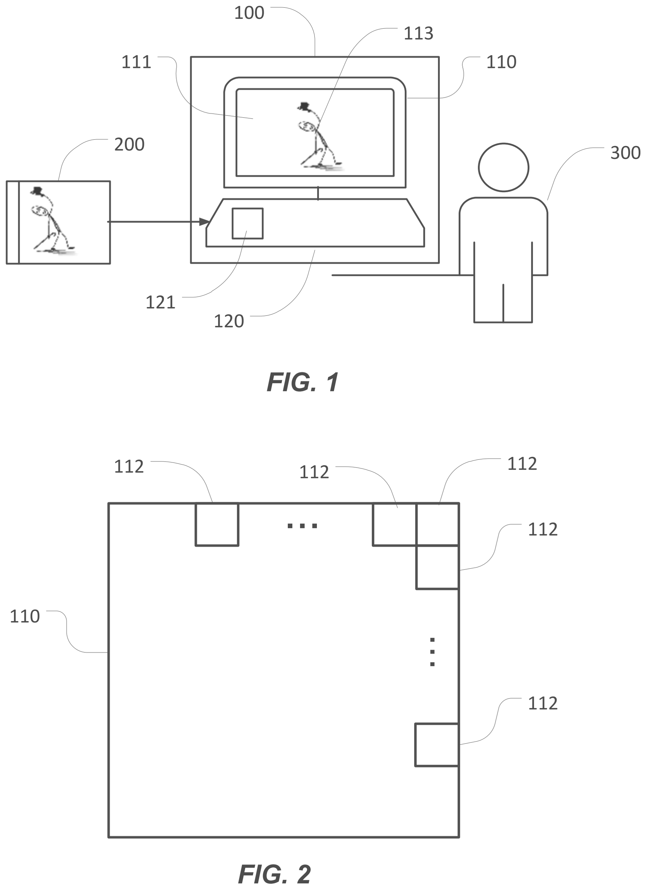

FIG. 1 schematically shows an embodiment of a phototherapeutic apparatus. The phototherapeutic apparatus, generally designated **100,** comprises a display device **110** and a control unit **120.**

The control unit **120** and the display device **110** may be embodied as a single device accommodated in a single housing or they may be implemented as separate devices communicatively coupled to each other.

The display device **110** defines a display area **111** for displaying one or more images **113** to be viewed by a user **300.** The display device may use any suitable display technology, such as an LED display, an OLED display, a Q-LED display, an LCD display.

The control unit **120** may be an integrated circuit or other suitable circuitry configured for controlling the display device. To this end, the control unit may implement a display controller.

The control unit **120** is configured to receive image data **200** and control the display device to display one or more images **113** responsive to the received image data. For example, the control unit may include a frame rate controller for controlling a frame refresh rate of the display device. The control unit further comprises a color-flicker controller for controlling the color flicker of individual pixels of the display device as described herein. The control unit may further comprise a luminance controller for controlling luminance of individual pixels. The luminance controller may e.g. be implemented as a PWM controller.

The image data **200** may be received from a device external to the apparatus **100** or it may be retrieved from a storage device of the apparatus **100** or it may be generated by a processing unit of the apparatus **100.** Accordingly the apparatus **100** may include a suitable communications interface for receiving image data, e.g. a wired or wireless data communications interface such as a network adapter, a wifi interface, a Bluetooth interface and/or the like. Alternatively or additionally, the apparatus **100** may include a data storage device, such as a hard disk, an EEPROM, or another suitable data storage device. Yet alternatively or additionally, the apparatus may include a processing unit, such as a CPU, a GPU and/or the like.

The image data **200** may be indicative of a single image or of multiple images, e.g. frames of a video. The image data may be encoded according to a suitable coding scheme for image data.

In the example of FIG. 1, the control unit **120** comprises a mapping module **121** for mapping color information onto sets of color components, as will be described in greater detail below. For example, the mapping module may include a look-up table for mapping color information onto sets of color components.

The apparatus **100** may include additional components, e.g. a loudspeaker and corresponding audio control circuit for rendering audio information.

FIG. 2 schematically shows a display of an embodiment of a phototherapeutic apparatus, e.g. the apparatus of FIG. 1. The display **110** comprises a matrix of individually controllable light sources, also referred to as pixels **112.** Each pixel is configured to emit colored light and the color and luminance of the individual pixels is controllable by the control unit of the phototherapeutic apparatus. Accordingly, the display emits a spatially resolved pattern of light, e.g. an image or other spatially varying pattern. To this end, an image to be displayed with pixel resolution NxM may be suitably encoded to include target image color information C(i,j), i = 1...N, j = 1...M for each pixel, where the pixels are indexed by a row index i and a column index j.

In the display **110,** all pixels may be color fusion pixels as described herein, i.e. all pixels may be configured to alternatingly emit at least respective first and second light, alternating at a color-flicker frequency; the first light having a first set of color components resulting in a first emitted color and the second light having a second set of color components resulting in a second emitted color; wherein the color-flicker frequency is selected high enough to cause the alternatingly emitted first and second light to be perceived by a human observer as light having a user-perceptible fused color. Alternatively, only a subset of pixels may be color fusion pixels, which still allows the apparatus to stimulate brain functions in a user's brain. As the pixels are quite small there will be, dependent on the display, a limit to the requirements of how large a subset of the pixels should be color fusion pixels in order to achieve a suitable stimulation, e.g. whether it should be every 2nd, 3rd, 4th etc. pixel that should be color-fused. In some embodiments, not all pixels that are operable as color fusion pixels are operated as color fusion pixels at all times. In some embodiments, the control unit of the apparatus may selectively select which pixels to operate as color fusion pixels, e.g. based on the desired strength of neural stimulation and/or responsive to the received image data to be displayed. In any event, it is preferred that all color fusion pixels of the display are controlled to operate synchronously, i.e. perform the color-flickering at the same color-flicker frequency and, preferably, at the same phase.

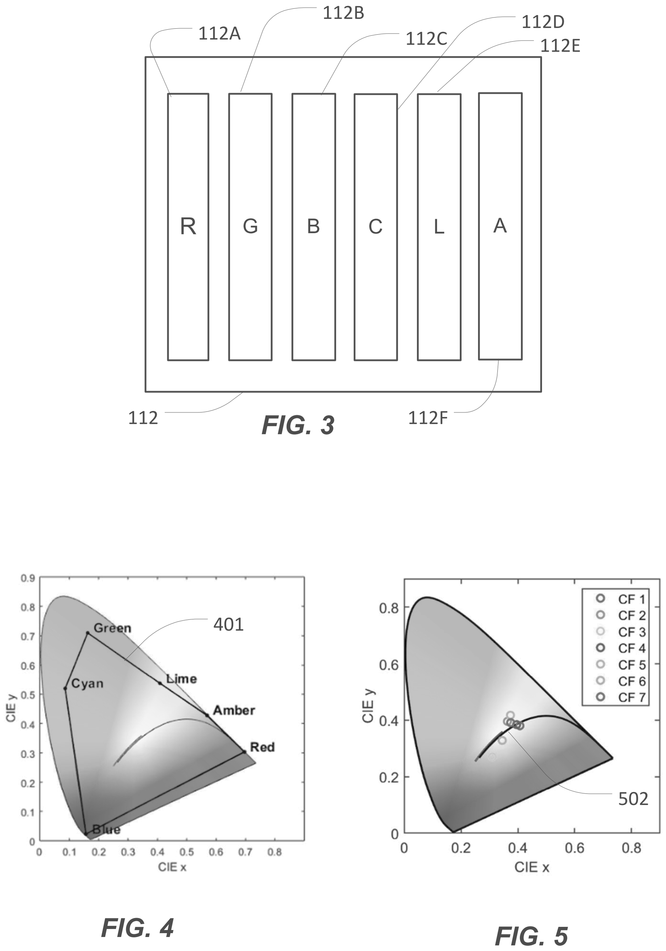

FIG. 3 schematically illustrates an embodiment of a pixel **112.** In the example of FIG. 3, the pixel is a multi-primary color pixel, which comprises six sub-pixels **112A-F.** Sub-pixel **112A** is operable to emit red light, sub-pixel **112B** is operable to emit green light and sub-pixel **112C** is operable to emit blue light, i.e. sub-pixels **112A-C** correspond to standard RGB color sub-pixels. Additionally, pixel **112** of the present embodiment includes sub-pixels having additional colors, in this example cyan sub-pixel **112D,** lime sub-pixel **112E** and amber sub-pixel **112F.** It will be appreciated that other examples of multi-primary color pixels may include additional or alternative color sub-pixels, e.g. yellow, magenta, white, etc. sub-pixels. It will further be appreciated that other examples of multi-primary color pixels may include a different number of color sub-pixels, such as 4, 5, 6, 7 or even more sub-pixels. Multi-primary color pixels allow the provision of displays that can reproduce a wider gamut color than conventional displays. Moreover, this additional variability has turned out to be of particular advantage when employing color fusion as described herein, since the additional sub-pixels allow a larger variability of selecting alternative sets of colors between which the pixel can alternate, so as to increase the range of displayable colors that the human eye can see. The pixel **112** can be controlled to selectively activate one or more of the sub-pixels at respective intensities/luminance, thereby causing the pixel to emit color light resulting from a mix of the individual colors of the sub-pixels. The intensities of the sub-pixels can e.g. be controlled by pulsed activation of the sub-pixels at variable duty cycles, e.g. by pulse-width modulation.

FIGs. 4 and 5 schematically illustrate color fusion with an embodiment of a phototherapeutic apparatus.

In particular, FIG. 4 illustrates an example of a color gamut **401** within which fused colors can be produced by a multi-primary color pixel including red, green, blue, lime cyan and, optionally, amber sub-pixels, e.g. as described in connection with FIG. 3. Colors within gamut **401** can be produced by controlling the pixel to alternatingly activate a first set of sub-pixels and a second set of sub-pixels at a suitable color-flicker frequency.

In particular when the color-flicker frequency is selected high enough, e.g. at least 25 Hz, the user viewing the display will perceive the emitted light by the pixel as having a fused color inside color gamut **401** rather than a pixel alternating/flickering between two different colors. Moreover, when the color-flicker frequency is suitably selected, the flickering can induce brain stimulating neural responses in the user's brain. Examples of suitable color-flicker frequencies, which result in imperceptible brain stimulation from the flickering light while the user perceives a fused color, include frequencies between 25 Hz and 60 Hz, such as between 30 Hz and 50 Hz, such as between 35 Hz and 45 Hz, such as between 38 Hz and 42 Hz, such as 40 Hz.

In preferred embodiments, in order for the alternating activation at the color-flicker frequency to remain substantially imperceptible for the user, it is preferred that the alternating sets of colors are emitted at substantially the same user-perceived luminance so as to avoid a user-perceivable luminance flickering, as most subjects are more sensitive to luminance flicker than to color flicker. The inventors have realized that this luminance matching of the alternating colors is very beneficial for ensuring that the user perceives a fused color without unduly perceiving a flickering light. However, the requirement of luminance matching imposes limitations of the range of fused colors a color fusion pixel may emit. For example, when continuously mixing two colors from two sub-pixels, i.e. by concurrently emitting light from said two sub-pixels, the whole line of colors between the monochromatic colors of the respective sub-pixels can be achieved by controlling the relative luminance of the two sub-pixels. However, when employing brain-stimulating color fusion by alternatingly activating two sub-pixels at a color-flicker frequency, in order to maintain luminance matching for the flicker of the color-fused light to appear substantially imperceptible, only a single color along the line between the two primary colors may be achievable rather than the whole line of colors. Accordingly, in order to maintain luminance matching for a color fusion pixel, one cannot simply adjust each set of colors independently from the other set of colors without reducing or even eliminating the effect of color fusion. Thus, imposing luminance matching imposes some restriction on the number of combinations possible.

For example, a conventional display pixel utilizing color mixing by simultaneously emitting multiple primary colors at respective luminance, a pixel having three sub-pixels (e.g. RGB pixels) may provide a 24-bit / (8+8+8)-bit true color RGB implementation where each sub-pixel has 256 discrete levels of luminance per channel. The maximum number of combinations of colors are thus 256x256x256 = 16.7 million colors. For a 48-bit / (16+16+16)-bit / deep color RGB implementation this is 65536x65536x65536 = 281475 billion colors. In comparison, a 16-bit RGB implementation is often referred to as high color with an 8-bit RGB implementation is just called color. The above calculation of the maximum number of possible color combinations in a conventional e.g., 16-bit RGB display assumes that each channel can be adjusted independently so as to generate a new color. For a color fusion pixel of embodiments of the apparatus described herein, imposing luminance matching implies that each channel cannot always be adjusted independently without having to adjust the other channels; otherwise the effect of color fusion may be reduced or even be eliminated. Thus, this puts some restriction on the number of possible combinations. This may be illustrated by an example of a limited color gamut with linear current driving: Assuming a particular color-fused light emitted by a color fusion pixel including three 8-bit sub-pixels (e.g. RGB), the pixel may be controlled to alternatingly emit two sets of colors - SET1 and SET2, respectively - where the two sets correspond to the following sub-pixel luminance values:
[20, 20, 20] SET 1
[0, 40, 0] SET 2

Assuming that it is desirable to reduce the overall luminance of the pixel without changing the displayed color, each individual luminance may be divided by a factor of 20 to reduce luminance of the pixel by a factor of 20:
[1, 1, 1] SET 1
[0, 2, 0] SET 2

Now, the pixel brightness cannot be reduced further without destroying the color of the light. In general, when 1 element goes to zero or to 257 the limits are reached. Thus, the full space of the 8-bit resolution cannot be used and will be color.

Typically, a color gamut will span an area of possible colors. However, for individual pixels with color fusion between two monochromatic sub-pixels, the color gamut spans a line. Adding additional sub-pixels (e.g. additional LEDs having different colors as in a multi-primary color display) allows the color gamut to move around the line, however, not infinitely since some combinations of sub-pixels may either destroy the luminance matching or the color changes due to the minimum and maximum limit of the individual pixel resolution, as illustrated by the above example. Alternatively or additionally, the color gamut may be expanded by operating a pixel to employ a combination of color mixing and color fusion, namely by selecting the first and second sets of colors to include one or more common color components and one or more different color components. In particular the second set of color components may include one or more color components also present in the first set of color components and the second set of color components may additionally include one or more color components that are not present in the first set of color components or present to a substantially lesser degree. Generally, the individual color components emitted by a light source may be defined by respective light-emitting sub-elements of said light source, i.e. each light source may comprise a plurality of sub-elements, in particular sub-pixels, each sub-element configured to emit a respective color components.

FIG. 5 illustrates examples of possible fused colors achievable with a multi-primary color pixel as described in FIG. 3. In particular, FIG. 5 illustrates that multiple different whites and colors around the Planckian locus **502** can be obtained.

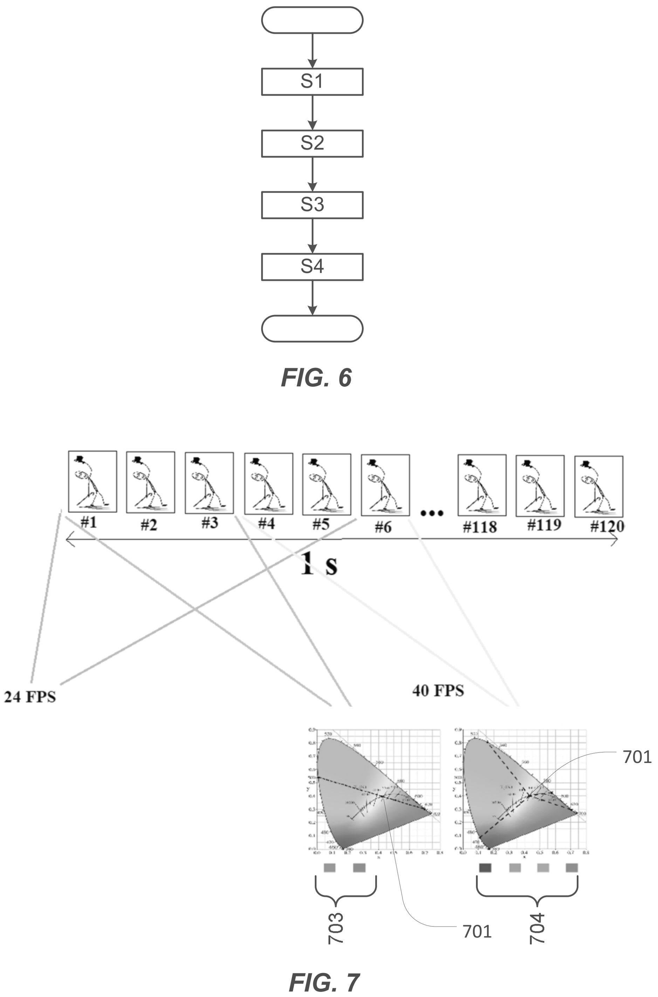

FIG. 6 schematically illustrates a process of controlling an embodiment of a phototherapeutic apparatus, e.g. the apparatus described with reference to FIGs. 1-3.

In initial step **S1,** the process receives image data. The image data may represent a single image or a sequence of images and be represented in any suitable form, e.g. as a suitable graphics format, such as jpg, or a suitable format for encoding media content such as mp4. The image data may represent an image in a suitable resolution and define color values for each of a matrix of image pixels.

Optionally, in step S2, the process determines which subset of display pixels are to be operated as color fusion pixels and which display pixels are to be operated as conventional display pixels, i.e. solely employing color mixing of concurrently emitted color components.

In particular, in some embodiments the process only selects display pixels that are to represent certain predetermined target image colors as color fusion pixels, while the remaining display pixels may be operated as conventional display pixels.

Alternatively or additionally, the process only selects display pixels as color fusion pixels that are to represent the same, or at least substantially the same, target image color for at least a minimum period of time, e.g. for at least 100 ms such as for at least 200 ms. This ensures that the color flickering with the corresponding color components persists long enough for the observer's brain to perceive the flickering colors as a fused color. Accordingly, when displaying a video or otherwise time-varying image data, the subset of display pixels that are operated as color fusion pixels may change over time.

In step **S3,** for each display pixel to be operated as a color fusion pixel, the process determines two sets of colors from the received image color for said display pixel, e.g. based on a color look-up table. It will be appreciated that, in embodiments where only a subset of the display pixels are color fusion pixels, this determination may only need to be performed for said subset. Alternatively, for the display pixels to be operated as conventional pixels, the process may select the two sets of colors to be identical with each other.

An example of a color look-up table may be indexed by a suitable color space representation, e.g. by chromaticity pair coordinates (x,y) which may each be suitably discretized parameters between 0 to 1.

For each chromaticity coordinate, the look-up table may define two or more sets of colors. Each set of colors may be represented as an array of intensity values. Each intensity value may represent one of the primary colors of the display, e.g. blue, cyan, green, lime, amber, red. Each intensity value may be defined at a suitable resolution e.g. as an 8-bit or 16 bit value.

For example, color look up table may be represented as a table, e.g. as in the following example:
x-y

| | | BC | G | L |
|---|---|---|---|---|
| Set1 | 0 | 10 | 10 | 15 |
| Set2 | 100 | 5 | 50 | 5 |

x-y

| | | BC | G | L |
|---|---|---|---|---|
| Set1 | 0 | 20 | 20 | 30 |
| Set2 | 200 | 10 | 100 | 10 |

Generally, a multi-primary color pixel with more than three primary colors (e.g. RGB and one, two or more further colors) is advantageous because it allows a larger number of fused color combinations while maintaining sufficient luminance matching.

In one example, a 6-color pixel is assumed (e.g. having ordinal values blue, cyan, green, lime, amber, red, respectively). However, a space can also be derived from a 3-color traditional RGB space or a different set of multi-primary colors and/or for a different resolution implementation. Such a space can be generated by calibration of the specific display technology by conducting all possible combinations of color-fusion pixels and measuring the perceived (x,y) pairs for all combinations of specific display at hand. It is noted that the color space does not have to be represented in the (x,y) space, but may be represented in a similar color space, e.g. in the (u,v) space, (u*,v*) or similar color space. It will thus be appreciated that other examples of look-up tables may be based on a different color space. Yet further, some embodiments of the apparatus may have stored thereon different look-up tables for different color spaces. Utilizing linear driving of the LEDs or other types of sub-pixels, and also having a well-defined maximum and minimum LED intensity value, the luminance value of each pixel is not necessary to measure in a calibration stage.

In step **S4,** the process controls the individual display pixels, or at least the subset to be operated as color fusion pixels, to alternatingly activate the sub-pixels according to the determined sets of colors.

The activation of the individual display pixels may be controlled based on multiple update rates:
1) The displayed image or images may be updated at a predetermined display refresh rate. The refresh rate determines the number of times per second the display can redraw the display area. The refresh rate is thus related to the maximum number of frames the display can display per second. Common display refresh rates include 120 Hz, 60 Hz and 30 Hz.
2) Each pixel may alternate between the determined two sets of colors at the color-flicker frequency. The refresh rate should preferably be at least twice the color-flicker frequency.
3) The luminance of the individual sub-pixels may be controlled by pulsed activation at a pulse rate, e.g. using pulse width modulation of the luminance. PWM may operate at a wide range of frequencies, e.g. PMW frequencies in the kHz or even MHz range. In some embodiments, PMW frequencies in the range between 100 Hz and 100kHz are preferred, but other frequencies may be used as well.

In some embodiments, the display refresh rate is higher than the color-flicker frequency and the pulse rate is higher than the frame refresh rate.

FIG. 7 illustrates an example of a display operating at a display refresh rate of 120 Hz and displaying a video stream having 24 fps, i.e. a new frame is displayed after every fifth display refresh cycle.

For each frame, each pixel displays a target color as defined by the image data for the particular frame. The display displays the target color of all or at least a subset of the pixels as a fused color, i.e., by alternating between two sets of primary colors at a color-flicker frequency. In the example of FIG. 7, the color-flicker frequency is 40 Hz, i.e., each pixel alternates between two sets of primary colors every three frames.

In FIG. 7, this is illustrated for a pixel displaying a fused color **701** close to the Planckian locus **702.** The fused color is displayed by alternatingly display a first set of primary colors **703** - in this example red (e.g., 624 nm) and blue (e.g. 500 nm) - and a second set of primary colors **704 -** in this example a set of four primary colors.

Luminance matching of the two sets of colors may be achieved by controlling the relative intensities of the primary colors, e.g., by PMW modulation, e.g. at 1500 kHz.

In the example of FIG. 7, the frame rate and the color-flicker frequency are such that a single flicker cycle is longer than the duration of a single frame. Accordingly, when the color of a display pixel changes rapidly, there is not sufficient time to complete one or more flicker cycles before the target image color for the particular pixel changes again. This may negatively impact the color perception by the observer, which may be mitigated, e.g. by suitable selection of the frame rate relative to the color-flicker frequency. Alternatively, the apparatus may adaptively select which pixels are to be operated as a color fusion pixel at any given time. In particular, based on the received stream of image data, the apparatus may selectively only operate pixels as color fusion pixels where the pixel color remains sufficiently constant for a sufficiently long period to allow multiple flicker cycles.

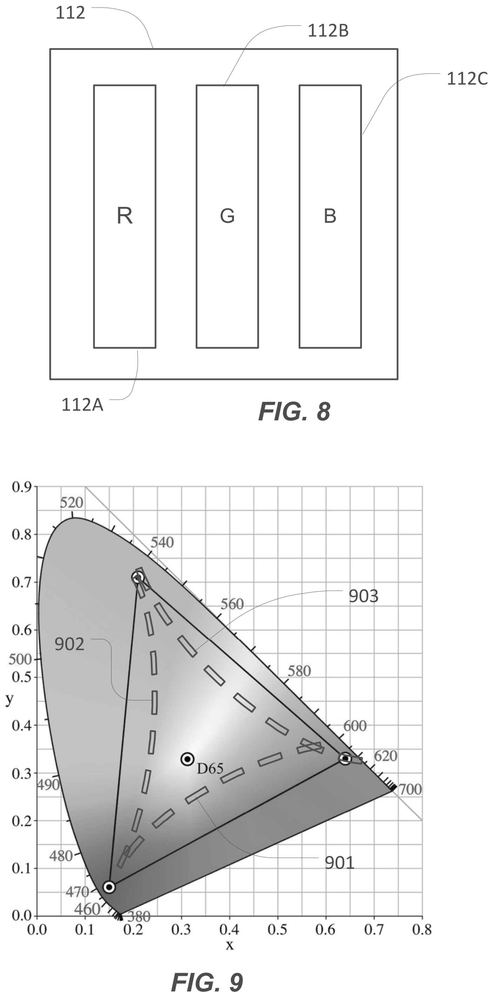

FIG. 8 schematically shows a light source of another embodiment of a phototherapeutic apparatus. In particular, FIG. 8 schematically illustrates an embodiment of a pixel **112.** In the example of FIG. 8, the pixel is an RGB pixel and comprises three sub-pixels **112A-C.** Sub-pixel **112A** is operable to emit red light, sub-pixel **112B** is operable to emit green light and sub-pixel **112C** is operable to emit blue light. The pixel **112** can be controlled to selectively activate one or more of the sub-pixels at respective intensities, thereby causing the pixel to emit color light resulting from a mix of the individual colors of the sub-pixels. The intensities of the sub-pixels can e.g. be controlled by pulsed activation of the sub-pixels at variable duty cycles, e.g. by pulse-width modulation.

FIGs. 9 - 10 schematically illustrate color fusion with an embodiment of a phototherapeutic apparatus, in particular an embodiment having color fusion pixels including three sub-pixels, e.g. as described in connection with FIG. 8.

For example, FIG. 9 illustrates how, though to a more limited extent, color fusion can be obtained even with three-sub-pixel pixels, e.g. RGB pixels. For example alternating between a first color set (R + B) with a second color set (G) will only allow colors under the Planckian curve to appear imperceptible, as illustrated by line **901** in FIG. 9. Similarly, color fusion of a first set (B + G) with a second set (R) will generate colors around a line **902.** Yet further, color fusion of a first set (R + G) with a second set (B) will generate colors around a line **903.**

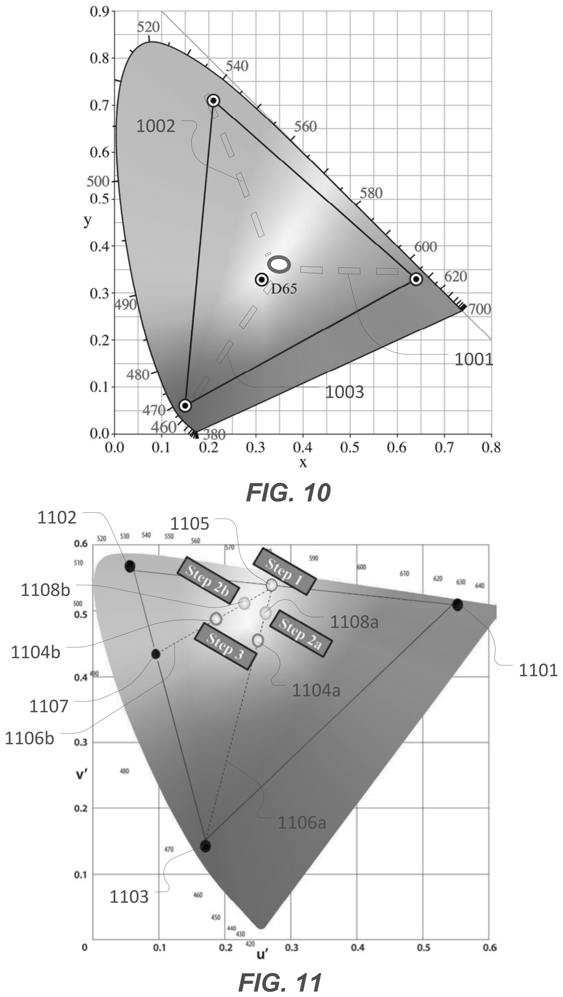

FIG. 10 illustrates a similar color fusion option where a first set includes components from all three primary colors (RGB) while the second set only includes either R (line **1001),** G (line **1002)** or B (line **1003).**

FIG. 11 illustrates an example of a process for obtaining a first and a second sect of color components for a target image color where from a color gamut spanned by three primary colors R **(1101),** G **(1102)** and B **(1103),** corresponding to a display having RGB light sources.

In particular, illustrates a first example, where an image target color 1104a is to be represented by an RGB display pixel, i.e. using only color components R, G, and B.

In an initial step 1, the process selects two of the primary color components. In the example of FIG. 11, the process has selected color components R and G. However, it will be appreciated that, alternatively, each of the pairs R+B or G+B may be selected instead. The process adds one of the selected color components, e.g. R, to the first set of color components, and the process adds the other of the selected color components, e.g. G, to the second set of color components. The process then determines the fused color 1105 obtainable from luminance-matched color fusion of the selected two color components. In particular, the process may determine the color coordinates of the fused color in a suitable color space. As explained above, by luminance-matched color fusion of the selected two color components, one fused color may be obtained.

In a subsequent step 2a, the process determines a further color component to be added to the first and second sets of color components. Dependent on the location of the target image color 1104a in the color space, the further color component may be the third primary color component, in this example B, or a mixed color including color component B and one of the color components R and G. In the first example illustrated in FIG. 11, the image target color 1104a is located such that the further color component is the third primary color B. By adding a suitable amount of component B to both sets of color components, the resulting color 1108a perceived by the viewer will lie on the dashed line 1106a connecting the fused color 1105 and the further color component 1103. Finally, in step 3, the process determines the relative intensity of the further color component 1103 so as to move the resulting perceived color 1108a along line 1106a until it coincides with the target image color 1104a.

Accordingly, the resulting first set of color components includes respective amounts of color components R and B, and the second set of color components includes respective amounts of the color components G and B. When the light source is controlled to alternate between the first and second set of color components at a suitable color-flicker frequency and determined relative intensities, the viewer perceives the emitted color as the intended target image color 1104a while not perceiving any substantial flicker. The viewer's color perception results from a combination of color fusion of the primary colors R and G combined with a color mixing of the fused color 1105 with the third primary color B. The process may thus store the determined intensities of color components in a look-up table indexed by the respective target image color.

FIG. 11 further illustrates a second example of a different target image color 1104b. In this case the further color component to be added to the first and second sets of color components (in step 2b) is itself a mixed color 1107, in this case resulting from mixing color components G and B at an appropriate ratio of intensities. Adding this mixed color 1107 (i.e. adding G and B color components in an appropriate ratio) to each of the first and second set of color components results in a perceived color 1108b along the dashed line 1106b. As in the first example, the process then adjusts the relative intensities of the further color components and the color components creating the fused color so as to align the perceived color 1108b with the target image color 1104b. Accordingly, the resulting first set of color components includes color components R, G and B, and the second set of color components includes the color components G and B in respective intensity ratios. As in the first example, the viewer's color perception results from a combination of color fusion of the primary colors R and G combined with a color mixing of the fused color 1105 with the further color 1107, which in this case itself is a mixed color.

Accordingly, in some embodiments, the first and second set of color components each comprise or consist of color components selected from a set of three primary color components, in particular from a red (R) color component, a green (G) color component and a blue (B) color component, and determining the first and second sets of color components that are alternatingly emitted by the light source representing a display pixel are determined by:
- adding a first primary color component of the set of three primary color components to the first set of color components and a second primary color component of the set of three primary color components, different from the first primary color component, to the second set of color components at respective amounts to enable luminance-matched color fusion of the first and second primary color components resulting in a perceived fused color,
- adding a supplementary set of one or two primary color components of the set of three primary color components to each of the first and second sets of color components, the supplementary set including a third primary color component of the set of three primary color components, different from the first and second primary color components, the one or two primary color components of the supplementary set having respective amounts selected such that a resulting color, resulting from concurrently emitting the one or two primary color components of the supplementary set, when concurrently emitted with the fused color, result in the target image color.

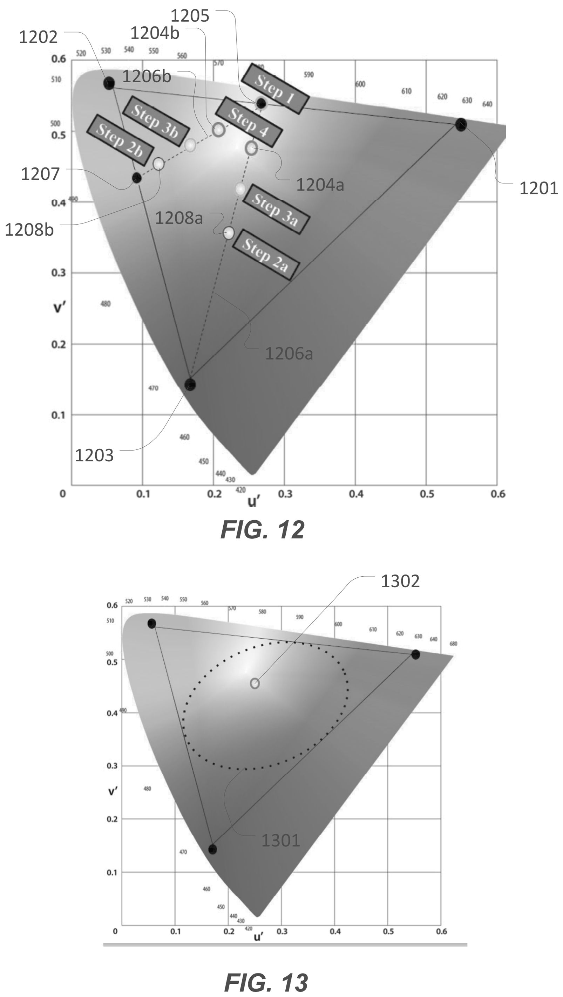

FIG. 12 illustrates another example of a process for obtaining a first and a second sect of color components for a target image color where from a color gamut spanned by three primary colors R **(1201),** G **(1202)** and B **(1203),** corresponding to a display having RGB light sources.

In particular, FIG. 12 illustrates a first example, where an image target color 1204a is to be represented by an RGB display pixel, i.e. using only color components R, G, and B.

In an initial step 1, the process selects two of the primary color components. In the example of FIG. 12, the process has selected color components R and G. However, it will be appreciated that, alternatively, each of the pairs R+B or G+B may be selected instead. The process determines a mixed color 1205 obtainable by static mixing (in particular by concurrently emission) of the selected color components. In a subsequent step 2a, the process adds the appropriate ratios of the selected color components to both the first and the second set of color components. The process then determines a fused color 1208a obtainable from luminance-matched color fusion of the mixed color 1205 and the third primary color component B. In particular, the process may determine the color coordinates of the fused color 1208a in a suitable color space. As explained above, by luminance-matched color fusion of the mixed color 1205 and the third primary color B, one fused color 1208a on the line 1106a may be obtained. The process adds the color component B at the intensity suitable for luminance-matched color fusion with the mixed color 1205 to the first set.

In a subsequent steps 3a and 4, the process adds or subtracts appropriate levels of color component B or of the combination of color components R+G simultaneously to/from both sets of color components so as to move the resulting perceived color along line 1106a to the intended target image color 1104a.

Accordingly, the resulting first set of color components includes respective amounts of color components R, G and B, and the second set of color components includes respective amounts the color components R and G (and optionally B, depending on the applicable compensation in step 3a). When the light source is controlled to alternate between the first and second set of color components at a suitable color-flicker frequency, the viewer perceives the emitted color as the intended target image color 1104a while not perceiving any substantial flicker. The viewer's color perception results from a combination of static color mixing of the primary colors R and G combined with a color floor fusion of the resulting mixed color 1205 with the third primary color B.

FIG. 12 further illustrates a second example of a different target image color 1104b. In this case, the further color component to be used for color fusion (in a corresponding step 2b replacing step 2a of the first example of FIG. 12) is itself a mixed color 1207, in this case resulting from static mixing respective amounts of color components G and B.

Accordingly, in some embodiments, the first and second set of color components each comprise or consist of color components selected from a set of three primary color components, in particular from a red (R) color component, a green (G) color component and a blue (B) color component, and determining the first and second sets of color components that are alternatingly emitted by the light source representing a display pixel are determined by:
- adding a first primary color component of the set of three primary color components and a second primary color component of the set of three primary color components, different from the first primary color component, to each of the first and second sets of color components at respective amounts to cause concurrent emission of the first and second primary color components to result in a mixed color;
- adding a supplementary set of one or two primary color components of the set of three primary color components to the first set of color components, the supplementary set including a third primary color component of the set of three primary color components, different from the first and second primary color components, the one or two primary color components of the supplementary set having respective amounts selected such that a resulting color, resulting from concurrently emitting the one or two primary color components of the supplementary set, when alternatingly emitted with the mixed color, enable luminance-matched color fusion of the resulting color and the mixed color to form a perceived fused color, wherein the fused color is located on a line in a color space spanned by the three primary color components, the line extending between the mixed color and the resulting color and intersecting the image target color;
- adding selected amounts of the supplementary set or of the mixed color to each of the first and second sets of color components, the selected amounts being selected such that the concurrent emission of the selected amounts and the fused color are perceivable as the target image color.

While the processes described with references to FIGs. 11 and 12 allow the generation of a broad range of target image colors using color fusion, optionally combined with static color mixing, based on three primary color components R,G and B, the color gamut obtainable by these processes, or otherwise, may be smaller than the entire RGB color gamut, in particular if a strong activation of therapeutically efficient neural activity is desired.

Firstly, the relative luminance of the alternating color components relative to the color components that are present in both sets and used for static color mixing, may result in the relative luminance of the flickering light to be so low that the strength of neural activation is reduced below a desirable level.

Secondly, the type of display may impose limitations on the resolution and relative intensities of the obtainable color values. For example, in many display systems, each of the RGB values may be set to integer numbers between 0 and 255, corresponding to an 8-bit encoding. As the human visual perception perceives some colors stronger than others, the luminance-matching of the color fusion imposes upper and lower boundaries of the obtainable color contents. This limitation may be mitigated by suitable design of the display, e.g. by adding additional blue sub-pixels so as to allow for higher blue luminance, and/or by adding sub-pixels emitting white light or other colors. Nevertheless, luminance matching of the color fusion flicker may impose a limitation on the obtainable color gamut for at least some display types.

FIG. 13 schematically illustrates an example of such a limited color gamut, where the obtainable colors, which may be represented using a sufficient amount of color fusion flickering to evoke therapeutically effective neural activity, are illustrated by dashed line 1301. Image target colors inside the dashed line 1301, e.g. target image color 1302, are image target color suitable for representation by color fusion flickering, optionally in combination with conventional color mixing as described above. Image target colors outside the area delimited by the dashed line 1301 are less suitable. Accordingly, as described herein, some embodiments, may select which image portions of an image are to be represented using color fusion responsive to the target image colors in the respective portions of the image to be displayed. Accordingly, the apparatus may select which display pixels are to be included in the first subset of image pixels to be controlled to alternatingly emit first and second light at a suitable color flicker frequency. It will be appreciated that the illustrated color gamut of FIG. 13 is merely a schematic illustration.

The exact shape and size of an obtainable color gamut depends on the specific display technology.

While the determination of the first and second sets of color components has been illustrated in the context of and RGB color gamut, i.e. for light sources having three sub-elements R, G, and B, respectively, it will be appreciated that first and second sets of color components may be determined in a similar manner for light sources having further sub-elements, e.g. light sources having R, G, B and white sub-elements, or other combinations of three, four or more sub-elements. Generally, some embodiments of the apparatus described herein are configured to represent at least some target image colors by a combination of color mixing of concurrently emitted colored light and color fusion of alternatingly emitted colored light, alternatingly emitted at the color-flicker frequency.

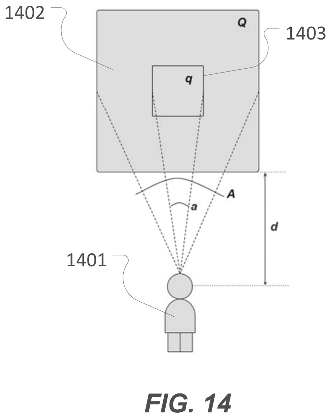

FIG. 14 schematically illustrates a display of an embodiment of a phototherapeutic apparatus. The display **1402** includes a display area Q which comprises a subarea q formed by one or more color fusion pixels. It will be appreciated that, in some embodiments, only a portion of the display area **1402** comprises color fusion pixels while the remaining portions may comprise conventional light-emitting pixels. In other embodiments, the entire display area **1402** comprises color fusion pixels. Also, for ease of illustration, FIG. 14 only shows a single subarea q with one or more color fusion pixels **1403.** It will be appreciated that embodiments of the phototherapeutic apparatus generally comprises a plurality of such pixels and may include one or more subareas with color fusion pixels. Moreover, again for purposes of easy illustration, the size of the area q with color fusion pixels **1403** relative to the area Q of the entire display area **1402** is merely an example. Generally, it will be appreciated that, in typical displays, the individual color fusion pixels are considerably smaller compared to the size of the entire display area.

In some embodiments, the size of the color fusion pixels and/or the part of the display area comprising color fusion pixels may be selected based on the intended viewing distance between the display area and a user **1401** viewing the display. During use of the phototherapeutic apparatus, the user is positioned a viewing distance *d* away from the display, thus resulting in the subarea *q* with one or more color fusion pixels **1403** being viewed under a viewing angle *a*. In some embodiments, the subarea area *q* with one or more color fusion pixels is selected such that, the viewing angle a is less or equal to the foveal retina field of view or approximately 3 degrees or less.

Generally, the further away the viewer is positioned, the larger the portion of the display area that includes color fusion pixels should be. In some embodiments, the apparatus may be configured to selectively control pixels as conventional pixels or as color fusion pixels. In some embodiments, the apparatus may include a distance sensor sensing the distance d between a user viewing the display and the display. The apparatus may then control the display responsive to the measured distance, e.g. by adjusting the number and/or positions of the pixels to be operated as color fusion pixels.

Embodiments of at least some of the method steps described herein may be computer-implemented. In particular, embodiments of some or all of the method steps disclosed herein may be implemented by means of hardware comprising several distinct elements, and/or at least in part by means of a suitably programmed microprocessor.

In the apparatus claims enumerating several means, several of these means can be embodied by one and the same element, component or item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, elements, steps or components but does not preclude the presence or addition of one or more other features, elements, steps, components or groups thereof.

Various embodiments disclosed herein may be summarized as follows:
Embodiment 1: A phototherapeutic apparatus comprising a plurality of light sources and a control circuit, the plurality of light sources being arranged in a spatial pattern so as to form at least a part of a display area of a display and each of the plurality of light sources being individually controllable to emit colored light; wherein the control circuit is configured to:
   - receive image data representing, for each light source of the plurality of light sources, a respective target image color to be represented by said light source;
   - control each light source of the plurality of light sources to emit colored light perceivable by a human observer as having the target image color to be represented by said light source;
   wherein the control circuit is configured, for each light source of at least a first subset of the plurality of light sources, to:
   - determine, responsive to the target image color to be represented by said light source, a first and a second set of color components; and to
   - control said light source to alternatingly emit at least respective first and second light, alternating at a color-flicker frequency, the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source.
Embodiment 2: A phototherapeutic apparatus according to embodiment 1; wherein the color-flicker frequency is selected for invoking a therapeutically effective neural response in the human observer's brain.
Embodiment 3: A phototherapeutic apparatus according to embodiment 1 or 2; wherein the color-flicker frequency is between 25 Hz and 60 Hz, such as between 30 Hz and 50 Hz, such as between 35 Hz and 45 Hz, such as between 38 Hz and 42 Hz, such as 40 Hz.
Embodiment 4: A phototherapeutic apparatus according to any one of the preceding embodiments; wherein the control circuit is configured to control the plurality of light sources so as to change the displayed image over time.
Embodiment 5: A phototherapeutic apparatus according to embodiment 4; wherein the control circuit is configured to control the plurality of light sources so as to cause a simulated movement of a first light-emitting area emitting light having a first user-perceptible target image color relative to a second light-emitting area emitting light having a second user-perceptible target image color.
Embodiment 6: A phototherapeutic apparatus according to embodiment 5; wherein the first and second user-perceptible target image colors are visually indistinguishable for a human observer but have different spectral distributions.
Embodiment 7: A phototherapeutic apparatus according to any one of the preceding embodiments; wherein the control circuit is configured to select, for each of the first subset of light sources and responsive to the received image data, the respective first and second color components such that the fused colors of the respective light sources of the first subset correspond to the respective target image colors.
Embodiment 8: A phototherapeutic apparatus according to any one of the preceding embodiments; wherein the control circuit is configured to cause the light sources of the first subset to synchronously alternate between the respective first and second sets of color components.
Embodiment 9: A phototherapeutic apparatus according to embodiment 8; wherein the control circuit is configured to cause all light sources of the plurality of light sources that are controlled to alternatingly emit respective first and second light to synchronously alternate between the respective first and second sets of color components.
Embodiment 10: A phototherapeutic apparatus according to any one of the preceding embodiments; wherein the control circuit is configured to determine the first and second sets of color components from a look-up table, the look-up table mapping target image colors to respective first and second sets of color components.
Embodiment 11: A phototherapeutic apparatus according to any one of the preceding embodiments; wherein the control circuit is configured to receive a stream of image data representing a video and to control the plurality of light sources to display the video within the display area.
Embodiment 12: A phototherapeutic apparatus according to any one of the preceding embodiments; wherein the control circuit is configured to only control the light sources of the first subset to alternatingly emit respective first and second sets of color components at the color-flicker frequency.
Embodiment 13: A phototherapeutic apparatus according to any one of the preceding embodiments; wherein the control circuit is configured to select the first subset of light sources responsive to the received image data.
Embodiment 14: A phototherapeutic apparatus according to embodiment 13, wherein the control circuit is configured to only include selected light sources of the plurality of light sources in the first subset, wherein the target image colors to be represented by the selected light sources belong to a predetermined set of target image colors.
Embodiment 15: A phototherapeutic apparatus according to embodiment 13 or 14, wherein the received image data is time varying image data representing, for each light source of the plurality of light sources, a series of respective target image colors; and wherein the control circuit is configured to change the first subset responsive to the time-varying image data.
Embodiment 16: A phototherapeutic apparatus according to embodiment 15, wherein the control circuit is configured to only include selected light sources of the plurality of light sources in the first subset, wherein the target image colors to be represented by the selected light sources persist for at least a minimum period of time, in particular at least 100 ms, such as at least 200 ms.
Embodiment 17: A phototherapeutic apparatus according to any one of the preceding embodiments; further comprising an audio output device and wherein the phototherapeutic apparatus is configured to output a sound signal modulated at said color-flicker frequency.
Embodiment 18: A phototherapeutic apparatus according to any one of the preceding embodiments; wherein the alternatingly emitted light at said color-flicker frequency is selected to stimulate or to entrain brain waves, in particular gamma oscillations, in a human's brain when the human is exposed to said alternatingly emitted light.
Embodiment 19: A phototherapeutic apparatus according to any one of the preceding embodiments; wherein the control circuit is configured to control each light source of at least the first subset of the plurality of light sources to emit the respective first and second light at substantially the same luminance.
Embodiment 20: A phototherapeutic apparatus according to embodiment 19; wherein the first and second sets of color components are selected to cause the alternatingly emitted first and second light to be perceived by a human observer as light having the image target color to be represented by said light source when the first and second light are emitted at substantially the same luminance.
Embodiment 21: A phototherapeutic apparatus according to any one of the preceding embodiments, wherein each light source comprises three or more light-emitting sub-elements configured to emit light having respective colors.
Embodiment 22: A phototherapeutic apparatus according to any one of the preceding embodiments, configured to represent at least some target image colors by a combination of color mixing of concurrently emitted colored light and color fusion of alternatingly emitted colored light, alternatingly emitted at the color-flicker frequency.
Embodiment 23: A phototherapeutic apparatus comprising a plurality of light sources and a control circuit, the plurality of light sources being arranged in a spatial pattern so as to form at least a part of a display area of a display and each of the plurality of light sources being individually controllable to emit colored light; wherein the control circuit is configured to:
   - control at least a first subset of the plurality of light sources to alternatingly emit respective first and second light, alternating at a color-flicker frequency, the first light having a first set of color components resulting in a first emitted color and the second light having a second set of color components resulting in a second emitted color;
   wherein the color-flicker frequency is selected high enough to cause the alternatingly emitted first and second light to be perceived by a human observer as light having a user-perceptible fused color.
Embodiment 24: The phototherapeutic apparatus according to any one of the preceding embodiments, wherein the color-flicker frequency is selected for invoking a therapeutically effective neural response in the human observer's brain.
Embodiment 25: The phototherapeutic apparatus according to any one of the preceding embodiments, wherein the phototherapeutic display apparatus is configured to emit the first and second light at substantially the same luminance.
Embodiment 26: The phototherapeutic apparatus according to any one of the preceding embodiments, wherein the color-flicker frequency of 40 Hz or between 38 Hz and 42 Hz.
Embodiment 27: The phototherapeutic apparatus according to any one of the preceding embodiments, further comprising an audio output device, and wherein the phototherapeutic apparatus is configured to output, via the audio output device, a sound signal modulated at said color-flicker frequency.
Embodiment 28: The phototherapeutic apparatus according to any one of the preceding embodiments, wherein the control circuit is configured to:
   - receive image data representing, for each light source of the plurality of light sources, a respective target image color to be represented by said light source;
   - control each light source of at least the first subset of the plurality of light sources to emit colored light perceivable by a human observer as having the target image color to be represented by said light source by alternatingly emitting at least respective first and second light, alternating at the color-flicker frequency, wherein the color-flicker frequency is selected high enough to cause the alternatingly emitted first and second light to be perceived by a human observer as light having the user-perceptible fused color, and wherein the fused color corresponds to the target image color to be represented by said light source.
Embodiment 29: A phototherapeutic apparatus comprising a plurality of light sources, an audio output device, and a control circuit, the plurality of light sources being arranged in a spatial pattern so as to form at least a part of a display area of a display and each of the plurality of light sources being individually controllable to emit colored light; wherein the control circuit is configured to:
   - receive image data representing, for each light source of the plurality of light sources, a respective target image color to be represented by said light source;
   - control each light source of at least a first subset of the plurality of light sources to emit colored light perceivable by a human observer as having the target image color to be represented by said light source by alternatingly emitting at least respective first and second light, alternating at a color-flicker frequency, wherein the color-flicker frequency is selected high enough to cause the alternatingly emitted first and second light to be perceived by a human observer as light having a user-perceptible fused color corresponding to the target image color to be represented by said light source;
   wherein the phototherapeutic apparatus is configured to output, via the audio output device, a sound signal modulated at said color-flicker frequency.
Embodiment 30: A phototherapeutic display apparatus comprising:
   - a plurality of light sources arranged in a spatial pattern so as to form at least a part of a display area of a display, each light source being individually controllable to emit colored light; and
   - a control circuit configured to control at least a first subset of the plurality of light sources to alternatingly emit respective first and second light, alternating at a color-flicker frequency of 40 Hz or between 38 Hz and 42 Hz, the first light having a first set of color components resulting in a first emitted color and the second light having a second set of color components resulting in a second emitted color;

   wherein the phototherapeutic display apparatus is configured to emit the first and second light at substantially the same luminance;
   wherein the color-flicker frequency is selected high enough to cause the alternatingly emitted first and second light to be perceived by a human observer as light having a user-perceptible fused color; and
   wherein the color-flicker frequency is selected for invoking a therapeutically effective neural response in the human observer's brain.
Embodiment 31: A method for controlling a phototherapeutic apparatus, the phototherapeutic apparatus comprising a plurality of light sources, the plurality of light sources being arranged in a spatial pattern so as to form at least a part of a display area of a display and each of the plurality of light sources being individually controllable to emit colored light, wherein the method comprises:
   - receiving image data representing, for each light source of the plurality of light sources, a respective target image color to be represented by said light source;
   - controlling each light source of the plurality of light sources to emit colored light perceivable by a human observer as having the target image color to be represented by said light source;
   wherein controlling comprises:
   - determining, responsive to the target image color to be represented by said light source, a first and a second set of color components; and
   - controlling said light source to alternatingly emit respective first and second light, alternating at a color-flicker frequency, the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source.
Embodiment 32: A method for invoking gamma waves in a subject's brain, the method comprising:
   - receiving image data representing, for each light source of a plurality of light sources, a respective target image color to be represented by said light source;
   - controlling each light source of the plurality of light sources to emit colored light perceivable by a human observer as having the target image color to be represented by said light source;
   wherein controlling comprises:
   - determining, responsive to the target image color to be represented by said light source, a first and a second set of color components; and
   - controlling said light source to alternatingly emit respective first and second light, alternating at a color-flicker frequency, the first light having the determined first set of color components resulting in a first emitted color and the second light having the determined second set of color components resulting in a second emitted color; wherein the color-flicker frequency is selected high enough to cause the alternatingly emitted first and second light to be perceived by a human observer as light having a user-perceptible fused color corresponding to the target image color to be represented by said light source and wherein the color-flicker frequency is selected for invoking gamma waves in the human observer's brain.
Embodiment 33: A method according to embodiment 31 or 32; wherein the first and second set of color components each comprise or consist of color components selected from a set of three primary color components, in particular from a red (R) color component, a green (G) color component and a blue (B) color component, and wherein determining comprises:
   - adding a first primary color component of the set of three primary color components to the first set of color components and a second primary color component of the set of three primary color components, different from the first primary color component, to the second set of color components at respective amounts to enable luminance-matched color fusion of the first and second primary color components resulting in a perceived fused color,
   - adding a supplementary set of one or two primary color components of the set of three primary color components to each of the first and second sets of color components, the supplementary set including a third primary color component of the set of three primary color components, different from the first and second primary color components, the one or two primary color components of the supplementary set having respective amounts selected such that a resulting color, resulting from concurrently emitting the one or two primary color components of the supplementary set, when concurrently emitted with the fused color, result in the target image color.
Embodiment 34: A method according to embodiment 31 or 32; wherein the first and second set of color components each comprise or consist of color components selected from a set of three primary color components, in particular from a red (R) color component, a green (G) color component and a blue (B) color component, and wherein determining comprises:
   - adding a first primary color component of the set of three primary color components and a second primary color component of the set of three primary color components, different from the first primary color component, to each of the first and second sets of color components at respective amounts to cause concurrent emission of the first and second primary color components to result in a mixed color;
   - adding a supplementary set of one or two primary color components of the set of three primary color components to the first set of color components, the supplementary set including a third primary color component of the set of three primary color components, different from the first and second primary color components, the one or two primary color components of the supplementary set having respective amounts selected such that a resulting color, resulting from concurrently emitting the one or two primary color components of the supplementary set, when alternatingly emitted with the mixed color, enable luminance-matched color fusion of the resulting color and the mixed color to form a perceived fused color, wherein the fused color is located on a line in a color space spanned by the three primary color components, the line extending between the mixed color and the resulting color and intersecting the image target color;
   - adding selected amounts of the supplementary set or of the mixed color to each of the first and second sets of color components, the selected amounts being selected such that the concurrent emission of the selected amounts and the fused color are perceivable as the target image color.

## Claims

1. A phototherapeutic apparatus comprising a plurality of light sources and a control circuit, the plurality of light sources being arranged in a spatial pattern so as to form at least a part of a display area of a display and each of the plurality of light sources being individually controllable to emit colored light; wherein the control circuit is configured to:
- control at least a first subset of the plurality of light sources to alternatingly emit respective first and second light, alternating at a color-flicker frequency, the first light having a first set of color components resulting in a first emitted color and the second light having a second set of color components resulting in a second emitted color;
wherein the color-flicker frequency is selected high enough to cause the alternatingly emitted first and second light to be perceived by a human observer as light having a user-perceptible fused color.

2. The phototherapeutic apparatus according to claim 1, wherein the color-flicker frequency is selected for invoking a therapeutically effective neural response in the human observer's brain.

3. The phototherapeutic apparatus according to claim 1 or 2, wherein the phototherapeutic display apparatus is configured to emit the first and second light at substantially the same luminance.

4. The phototherapeutic apparatus according to any one of the preceding claims, wherein the color-flicker frequency of 40 Hz or between 38 Hz and 42 Hz.

5. The phototherapeutic apparatus according to any one of the preceding claims, further comprising an audio output device, and wherein the phototherapeutic apparatus is configured to output, via the audio output device, a sound signal modulated at said color-flicker frequency.

6. The phototherapeutic apparatus according to any one of the preceding claims, wherein the control circuit is configured to:
- receive image data representing, for each light source of the plurality of light sources, a respective target image color to be represented by said light source;
- control each light source of at least the first subset of the plurality of light sources to emit colored light perceivable by a human observer as having the target image color to be represented by said light source by alternatingly emitting at least respective first and second light, alternating at the color-flicker frequency, wherein the color-flicker frequency is selected high enough to cause the alternatingly emitted first and second light to be perceived by a human observer as light having the user-perceptible fused color, and wherein the fused color corresponds to the target image color to be represented by said light source.

7. A phototherapeutic apparatus according to any one of the preceding claims; wherein the control circuit is configured to control the plurality of light sources so as to display an image and to change the displayed image over time.

8. A phototherapeutic apparatus according to claim 7; wherein the control circuit is configured to control the plurality of light sources so as to cause a simulated movement of a first light-emitting area emitting light having a first user-perceptible target image color relative to a second light-emitting area emitting light having a second user-perceptible target image color.

9. A phototherapeutic apparatus according to claim 8; wherein the first and second user-perceptible target image colors are visually indistinguishable for a human observer but have different spectral distributions.

10. A phototherapeutic apparatus according to any one of the preceding claims; wherein the control circuit is configured to select, for each of the first subset of light sources and responsive to received image data, the respective first and second color components such that the fused colors of the respective light sources of the first subset correspond to the respective target image colors.

11. A phototherapeutic apparatus according to any one of the preceding claims; wherein the control circuit is configured to cause the light sources of the first subset to synchronously alternate between the respective first and second sets of color components.

12. A phototherapeutic apparatus according to claim 11; wherein the control circuit is configured to cause all light sources of the plurality of light sources that are controlled to alternatingly emit respective first and second light to synchronously alternate between the respective first and second sets of color components.

13. A phototherapeutic apparatus according to any one of the preceding claims; wherein the control circuit is configured to determine the first and second sets of color components from a look-up table, the look-up table mapping target image colors to respective first and second sets of color components.

14. A phototherapeutic apparatus according to any one of the preceding claims; wherein the control circuit is configured to receive a stream of image data representing a video and to control the plurality of light sources to display the video within the display area.

15. A phototherapeutic apparatus according to any one of the preceding claims; wherein the control circuit is configured to select the first subset of light sources responsive to received image data.
